(19) Europäisches Patentamt
European Patent Office
Office européen des brevets

(11) Veröffentlichungsnummer: **0 515 876 A2**

# (12) EUROPÄISCHE PATENTANMELDUNG

(21) Anmeldenummer: **92107673.3**

(51) Int. Cl.[5]: **C07D 405/06**

(22) Anmeldetag: **07.05.92**

(30) Priorität: **31.05.91 DE 4117877**

(43) Veröffentlichungstag der Anmeldung:
**02.12.92 Patentblatt 92/49**

(84) Benannte Vertragsstaaten:
**AT BE CH DE DK ES FR GB GR IT LI NL PT SE**

(71) Anmelder: **BASF Aktiengesellschaft**
**Carl-Bosch-Strasse 38**
**W-6700 Ludwigshafen(DE)**

(72) Erfinder: **Seele, Rainer, Dr.**
**Leiblstrasse 3**
**W-6701 Fussgoenheim(DE)**
Erfinder: **Eicken, Karl, Dr.**
**Am Huettenwingert 12**
**W-6706 Wachenheim(DE)**
Erfinder: **Hickmann, Eckhard, Dr.**
**Kantstrasse 23**
**W-6701 Dannstadt-Schauernheim(DE)**
Erfinder: **Keil, Michael, Dr.**
**Fontanestrasse 4**
**W-6713 Freinsheim(DE)**
Erfinder: **Kober, Reiner, Dr.**
**Im Schlittweg 20**
**W-6701 Fussgoenheim(DE)**

## (54) Verfahren zur Herstellung von cis-2-(1H-1,2,4-Triazol-1-yl-methyl)-2-(halogenphenyl)-3-(halogenphenyl)-oxiran.

(57) Verfahren zur Herstellung von cis-2-(1H-1,2,4-Triazol-1-yl-methyl)-2-(halogenphenyl)-3-(halogenphenyl)-oxiran der allgemeinen Formel I

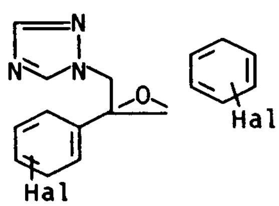

I,

in welcher Hal gleich oder verschieden ist und jeweils Fluor, Chlor oder Brom bedeutet, dadurch gekennzeichnet, daß man entweder

a) eine Carbonylverbindung der Formel II

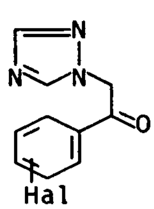

II

in welcher Hal für Fluor, Chlor und Brom steht, in Gegenwart einer Base mit einem Sulfoniumsalz der Formel VII

EP 0 515 876 A2

Rank Xerox (UK) Business Services

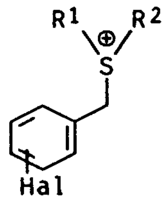

VII

in welcher Hal die oben angegebene Bedeutung hat und $R^1$ und $R^2$ unabhängig voneinander Alkyl, Cycloalkyl, einen Benzylrest, der im Phenylkern in gleicher Weise halogensubstituiert ist wie der bereits vorhandene Benzylrest in VII oder einen aromatischen Rest bedeuten, oder gemeinsam einen gesättigten oder ungesättigten 5- oder 6-gliedrigen Ring bilden, wobei das Sulfoniumsalz aus einem Benzylhalogenid der Formel III

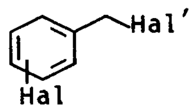

III,

in der Hal die o.g. Bedeutung hat und Hal' für Chlor, Brom oder Iod steht, und einem Sulfid der Formel IV

$R^1$-S-$R^2$ IV

gebildet wird, in Gegenwart oder Abwesenheit eines inerten Lösungsmittels zum Oxiran I umsetzt oder
b) einen Benzaldehyd der Formel VI

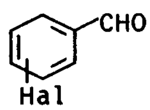

VI,

in der Hal die o.g. Bedeutung hat, in Gegenwart einer Base mit einem Sulfoniumsalz der Formel VIII

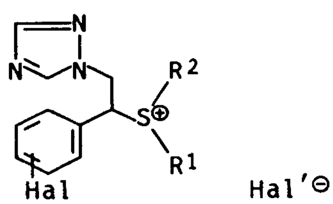

VIII,

in welcher Hal, Hal', $R^1$ und $R^2$ die o.g. Bedeutung haben, das aus dem entsprechenden Benzylhalogenidderivat der Formel V

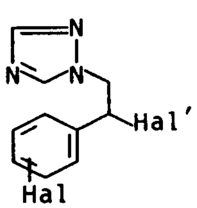

V,

in welcher Hal und Hal' jeweils die oben angegebene Bedeutung haben, und dem Sulfid IV gebildet wird, in Gegenwart oder Abwesenheit eines inerten Lösungsmittels zum Oxiran I umsetzt.

Die vorliegende Erfindung betrifft ein vorteilhaftes Verfahren zur Herstellung von cis-2-(1H-1,2,4-Triazol-1-yl-methyl)-2-(halogenphenyl)-3-(halogenphenyl)-oxiran der Formel I

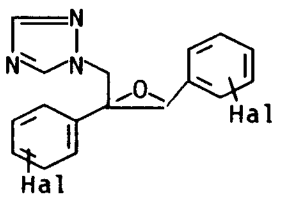

I,

in welcher Hal für Fluor, Chlor oder Brom steht.

Die Herstellung von Epoxiden der Struktur I ist z.B. in den deutschen Offenlegungsschriften 32 18 129 und 32 18 130 sowie in EP-A-196 038 beschrieben. Danach werden sie aus Halogenmethyloxiranen der Formel IX

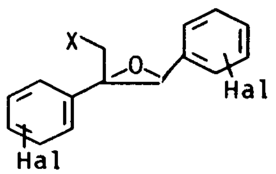

IX

in welcher X eine nukleophil substituierbare Abgangsgruppe (z.B. Cl oder Br) bedeutet und Hal die oben angegebene Bedeutung hat, durch Umsetzung mit Triazol erhalten. Die hierbei verwendeten Halogenmethyloxirane der Formel IX werden z.B. durch Oxidation von Diarylallylhalogeniden erhalten. Nachteilig an den Methoden des Standes der Technik sind die Verwendung teurer Reagenzien, z.B. teurer Halogenierungsreagenzien wie N-Bromsuccinimid für die radikalische Bromierung, die geringe Stereoselektivität und insbesondere die hohe Anzahl von Synthesestufen.

Es ist allgemein bekannt, daß sich Benzaldehyde unter basischen Reaktionsbedingungen mit verschiedenen Sulfoniumsalzen stereoselektiv in transkonfigurierte Diaryloxirane überführen lassen (siehe z.B. A.C. Knipe in The Chemistry of the Sulphonium Group, ed. C.J. M. Stirling and S. Patai, John Wiley & Sons Ltd., 1981, S. 313-374, insbesondere S. 359-363; L. Breau et al., Tetrahedron Lett., Band 31, S. 35-38 (1990); N. Furukawa et al., J. Org. Chem., Band 54, S. 4222-4224 (1989). Diese Diaryloxirane tragen jedoch keine weiteren sterisch anspruchsvollen Substituenten.

Moleküle, die in spezifischer Weise biologisch oder pharmakologisch wirken, müssen in vielen Fällen definierte geometrische Anordnungen bestimmter funktioneller Gruppen besitzen. Bei den fungiziden Wirkstoffen der Formel I (siehe DE-A-26 52 313) sind es vor allem die Z-konfigurierten Verbindungen (vgl. Sequenzregel nach Cahn, Ingold, Prelog), d.h. die Verbindungen, in denen die gegebenenfalls substituierten Phenylreste trans zueinander stehen, welche eine besonders hohe Wirksamkeit als Pflanzenschutzmittel besitzen.

Der Erfindung lag daher die Aufgabe zugrunde, ein Verfahren zu finden, nach welchem die Azolylmethyloxirane I in möglichst isomerenreiner Form, d.h. mit hoher Bevorzugung der trans-Konfiguration der Phenylreste am Oxiranring hergestellt werden können. Weiterhin bestand die Aufgabe, unter Verwendung vorteilhafter Zwischenprodukte und Vermeidung des Einsatzes von gefährlichen Peroxiden ein Herstellverfahren für die fungiziden Azolylmethyloxirane I zu finden, das sich gegenüber den in DE-A-32 18 129 und 32 18 130 beschriebenen Verfahren durch eine verkürzte Anzahl von Reaktionsschritten auszeichnet.

Demgemäß wurde ein Verfahren zur Herstellung von cis-2-(1H-1,2,4-Triazol-1-yl-methyl)-2-(halogenphenyl)-3-(halogenphenyl)-oxiran der allgemeinen Formel I

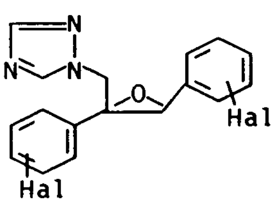

I,

in welcher Hal gleich oder verschieden jeweils Fluor, Chlor oder Brom bedeutet, gefunden, das dadurch gekennzeichnet ist, daß man entweder

a) eine Carbonylverbindung der Formel II

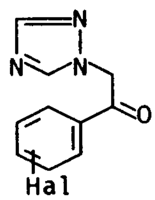

II

in welcher Hal für Fluor, Chlor und Brom steht, in Gegenwart einer Base mit einem Sulfoniumsalz der Formel VII

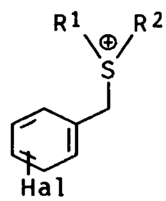

VII

in welcher Hal die oben angegebene Bedeutung hat und $R^1$ und $R^2$ unabhängig voneinander Alkyl, Cycloalkyl, einen Benzylrest, der im Phenylkern in gleicher Weise halogensubstituiert ist wie der bereits vorhandene Benzylrest in VII oder einen aromatischen Rest bedeuten, oder gemeinsam einen gesättigten oder ungesättigten 5- oder 6-gliedrigen Ring bilden, wobei das Sulfoniumsalz
aus einem Benzylhalogenid der Formel III

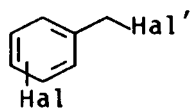

III,

in der Hal die o.g. Bedeutung hat und Hal' für Chlor, Brom oder Iod steht, und einem Sulfid der Formel IV

$R^1$-S-$R^2$      IV

gebildet wird, in Gegenwart oder Abwesenheit eines inerten Lösungsmittels zum Oxiran I umsetzt oder

b) einen Benzaldehyd der Formel VI

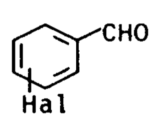

VI,

in der Hal die o.g. Bedeutung hat, in Gegenwart einer Base mit einem Sulfoniumsalz der Formel VIII

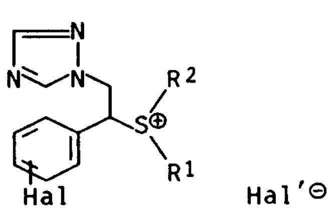

VIII,

in welcher Hal, Hal', $R^1$ und $R^2$ die o.g. Bedeutung haben, das aus dem entsprechenden Benzylhalogen-

idderivat der Formel V

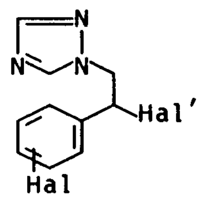

V,

in welcher Hal und Hal' jeweils die oben angegebene Bedeutung haben, in Gegenwart oder Abwesenheit eines inerten Lösungsmittels zum Oxiran I umsetzt.

Die Carbonylverbindungen der Formel II sind bekannt oder können in bekannter Weise, z.B. wie in DE-A-24 31 407 beschrieben, hergestellt werden. Die halogensubstituierten Benzaldehyde VI sind allgemein bekannt und z. T. käuflich zu erwerben.

Die Umsetzung dieser Carbonylverbindungen mit den Sulfoniumsalzen VII bzw. VIII wird in Gegenwart einer Base und im allgemeinen in Gegenwart eines Lösungs- oder Verdünnungsmittels vorgenommen.

Als Basen kommen organische oder anorganische Basen mit einem $pK_b$-Wert von > 10 in Betracht. Beispielsweise seien Alkalimetallhydroxide wie Lithium-, Natrium- oder Kaliumhydroxid; Alkalimetallcarbonate wie Natriumcarbonat, Kaliumcarbonat, Natriumhydrogencarbonat und Kaliumhydrogencarbonat genannt. Es können aber auch andere übliche Basen wie Alkalihydride, Alkaliamide und insbesondere Alkoholate, z.B. Alkalimetallalkoholate wie Natriummethylat, Natrium- oder Kalium-tert.-butylat sowie Lithiumalkylverbindungen, z.B. Methyllithium oder Butyllithium verwendet werden.

Die Basenmengen liegen üblicherweise bei 2 bis 5, insbesondere 2 bis 2,5 Moläquivalenten, bezogen auf das Sulfoniumsalz VII bzw. VIII.

Die Deprotonierung wird in der Regel bei Temperaturen von -50 bis 100 °C, vorzugsweise zwischen -30 und 50 °C, besonders bevorzugt bei -15 bis +20 °C, drucklos oder unter Druck kontinuierlich oder diskontinuierlich durchgeführt.

Zu den bevorzugten Lösungs- und Verdünnungsmitteln gehören polar aprotische Lösungsmittel z.B. Nitrile wie Acetonitril oder Propionitril, Ether wie Tetrahydrofuran, Diethylether, Ethylenglykoldimethylether, Dioxan oder Diisopropylether, Amide wie Dimethylformamid, Dimethylacetamid oder N-Methylpyrrolidon, ferner Dimethylsulfoxid oder entsprechende Gemische. Als Lösungsmittel kann auch die Sulfidkomponente IV verwendet werden.

Zweckmäßig verwendet man das Lösungsmittel in einer Menge von 50 bis 2000 Gew.-%, vorzugsweise 100 bis 1000 Gew.-%, bezogen auf die Carbonylverbindungen II bzw. VI.

Im allgemeinen wird die Carbonylverbindung II bzw. VI in einer Menge von 0,5 bis 3, insbesondere 1 bis 1,5 Moläquivalenten, bezogen auf das Sulfoniumsalz VII bzw. VIII eingesetzt.

Nach dem erfindungsgemäßen Verfahren werden die Sulfoniumsalze VII oder VIII aus den Benzylhalogenidderivaten III bzw. V durch Umsetzung mit den Sulfiden IV hergestellt.

Als Benzylhalogenide können die Chloride, Bromide und besonders bevorzugt die reaktiven Jodide eingesetzt werden. Die Benzylhalogenide der Formel III und V sind bekannt oder können in bekannter Weise gemäß DE-A-25 47 954 hergestellt werden.

Die Reste $R^1$ und $R^2$ in der Sulfidkomponente IV können breit variiert werden. Beispielsweise kommen verzweigte oder unverzweigte Alkylreste mit 1 bis 12, insbesondere 1 bis 6 Kohlenstoffatomen, Cycloalkylreste mit 3 bis 8 Kohlenstoffatomen, insbesondere Cyclohexyl- oder Cyclopentylreste oder aromatische Reste z.B. Phenylreste in Betracht. Die genannten Reste können noch unter den Reaktionsbedingungen inerte Substituenten tragen wie $C_1$-$C_4$-Alkyl oder $C_3$-$C_6$-Cycloalkyl. $R^1$ und/oder $R^2$ kann auch ein Benzylrest sein, der im Phenylrest in gleicher Weise halogensubstituiert ist wie der bereits vorhandene Benzylreste in VII. Die Reste $R^1$ und $R^2$ können ferner gemeinsam mit dem Schwefelatom, an das sie gebunden sind, einen 5- oder 6-gliedrigen Ring bilden, der ein oder zwei ethylenische Doppelbindungen enthalten kann oder vollkommen gesättigt ist, z.B. Tetrahydrothiofuranyl, Tetrahydrothiopyranyl, 2,3-Dihydrofuranyl, 2,3-Dihydropyranyl.

Beispielsweise seien folgende Sulfide genannt:

Dialkylsulfide wie Dimethylsulfid, Diethylsulfid, tert.-Butylmethylsulfid, Benzylsulfide wie Benzylmethylsulfid, 2-Chlorbenzylmethylsulfid, Dibenzylsulfid, Benzylphenylsulfid, cyclische Sulfide wie Tetrahydrothiofuran, Tetrahydrothiopyran, 2,2,5,5-Tetramethyltetrahydrothiofuran.

Zweckmäßigerweise wird man billige, leicht verfügbare Sulfide verwenden, die gut aus dem Reaktionsgemisch abtrennbar sind wie z.B. Dimethylsulfid.

Die Sulfidkomponente VI kann in stöchiometrischer Menge oder im überschuß, bezogen auf das Benzylhalogenid III oder V eingesetzt werden. Bevorzugt sind Mengen von etwa 1 bis 4 Moläquivalenten. Es ist auch möglich, die Sulfidkomponente als Lösungsmittel zu verwenden.

Bei Umsetzung des sekundären Halogenids V mit dem Sulfid IV ist es besonders vorteilhaft, wenn man dem Reaktionsgemisch Verbindungen hinzufügt, die das bei der nukleophilen Substitution austretende Halogenidion $Hal'^{\ominus}$ abfangen und durch Beeinflussung des Reaktionsgleichgewichts reaktionsbeschleunigend und ausbeutesteigernd wirken. Als Reaktionsbeschleuniger kommen vorzugsweise Salze von Schwermetallen, z.B. Quecksilber-, Zink- und Eisenhalogenide wie $FeCl_3$, $HgJ_2$ in Frage (s. hierzu J. Goedeler in Houben-Weyl, Methoden der Organischen Chemie Bd. IX, S. 171-194, insbesondere S. 179, Georg Thieme Verlag Stuttgart 1955). Es können aber auch Silbersalze wie $Ag_2CO_3$ oder $AgClO_4$ verwendet werden.

Es ist natürlich auch möglich, diese Halogenidionenfänger bei der Umsetzung von III mit IV hinzuzufügen.

Bei Zugabe solcher Reaktionsbeschleuniger ändert sich das Gegenion der Sulfoniumsalze VII und VIII je nach Art des zugesetzten Salzes, z.B. bei Zugabe von $FeCl_3$ in $[FeCl_4]^{\ominus}$ oder bei Zugabe von $HgJ_2$ in $[HgJ_3]^{\ominus}$. Der Reaktionsbeschleuniger wird bevorzugt nach dem Halogenatom des Benzylhalogenids III bzw. V ausgewählt.

Die Menge des Reaktionsbeschleunigers liegt vorzugsweise bei 0,5 bis 5, insbesondere 1 bis 1,5 Moläquivalenten, bezogen auf das Benzylhalogenid III bzw. V.

Die Herstellung der Sulfoniumsalze VII bzw. VIII kann in Abwesenheit oder vorzugsweise in Gegenwart eines Lösungsmittels bei Temperaturen von 0 bis 120, insbesondere 20 bis 100 ° C, vorgenommen werden. Als Lösungsmittel kommen ebenfalls polar aprotische Lösungsmittel in Frage wie vorstehend für die Umsetzung mit den Carbonylverbindungen aufgeführt.

Die dargestellten Sulfoniumsalze VII bzw. VIII können vorteilhaft direkt mit den Carbonylverbindungen II bzw. VI zu den Azolylmethyloxiranen I umgesetzt werden. Es ist ebenfalls möglich, insbesondere im Fall der sehr stabilen und gut kristallisierenden Salze VII, diese zu isolieren und anschließend gegebenenfalls unter Variation des Lösungsmittels in Gegenwart einer Base mit den Carbonylverbindungen umzusetzen.

Besonders bevorzugt ist die Verfahrensvariante, ein Gemisch der Carbonylverbindung II bzw. VI und dem Benzylhalogenid III bzw. V im Lösungsmittel zu dem in diesem Lösungsmittel gelösten Sulfid IV in Gegenwart der Base hinzuzufügen.

Es ist ferner möglich, die Sulfoniumsalze VII in einem inerten Lösungsmittel herzustellen, zunächst mit der Base zu deprotonieren und anschließend die Carbonylverbindung II hinzuzugeben. Im Fall der Verfahrensvariante b) geht man vorteilhaft in der Weise vor, daß man die Ausgangsverbindungen IV und V im Lösungsmittel vorlegt, das Reaktionsgemisch auf Temperaturen von 0 bis 120 ° C, vorteilhaft 20 bis 100 ° C, insbesondere 40-80 ° C erwärmt zur Bildung des Sulfoniumsalzes VIII, dieses anschließend mittels der Base deprotoniert und zuletzt den Benzaldehyd VI zusetzt.

Eine weitere mögliche Verfahrensvariante besteht darin, das Sulfoniumsalz VII und die Carbonylverbindung II im Lösungsmittel vorzulegen und zuletzt die Base hinzuzufügen, oder aber die Ausgangsstoffe IV und V zur Bildung des Sulfoniumsalzes VIII und dem Benzaldehyd V im Lösungsmittel vorzulegen und dann die Base hinzuzufügen.

Die Isolierung der Azolylmethyloxirane I aus dem rohen Reaktionsgemisch erfolgt in an sich bekannter Weise, z.B. durch Zugabe von Wasser und anschließender Extraktion mit einem organischen Lösungsmittel wie Methyl-tert.-butylether oder Dichlormethan zur Reinigung des erhaltenen Rohproduktes erfolgt eine Chromatographie an Kieselgel oder eine Umkristallisation in organischen Lösungsmitteln wie Ether, Ester oder Alkohole.

Die einzelnen Synthesewege sind in den nachfolgenden Beispielen dargestellt.

Beispiel 1 - Verfahrensvariante a)

cis-2-(1H-1,2,4-Triazol-1-yl-methyl)-2-(4-fluorphenyl)-3-(2-chlor-phenyl)-oxiran

Eine Lösung von 3,6 g (0,058 mol) Dimethylsulfid in 100 ml Acetonitril wird mit 10 g Kaliumhydroxid versetzt und anschließend bei 0 ° C ein Gemisch aus 86 g (0,053 mol) 2-Chlorbenzylchlorid und 10 g (0,049 mol) 2-(1H-1,2,4-Triazol-1-yl)-4'-fluor-acetophenon in 50 ml Acetonitril langsam zugetropft. Nachdem das Reaktionsgemisch zwei Stunden bei Raumtemperatur gerührt worden war, wird das Kaliumhydroxid abfiltriert, der Lösung 100 ml Wasser zugesetzt und mehrmals mit Methyl-tert.-butylether ausgeschüttelt. Die organische Phase wird mit Wasser gewaschen, über Natriumsulfat getrocknet und eingeengt. Der verbleibende Rückstand wird einer Chromatographie an Kieselgel mit n-Hexan/Essigester = 9:1 unterworfen, wobei 2,6 g (16 %) cis-2-(1H-1,2,4-Triazol-1-yl-methyl)-2-(4-fluorphenyl)-3-(2-chlorphenyl)-oxiran erhalten

werden.

Beispiel 2 - Verfahrensvariante b)

cis-2-(1H-1,2,4-Triazol-1-yl-methyl)-2-(4-fluorphenyl)-3-(2-chlor-phenyl)-oxiran

Eine Lösung von 2 g (0,007 mol) 1-Brom-1-(4-fluorphenyl)-2-(1H-1,2,4-triazol-1-yl)-ethan und 0,5 g (0,008 mol) Dimethylsulfid in 40 ml Acetonitril wird für zwei Stunden bei Raumtemperatur gerührt und anschließend für 30 Minuten auf 60° C erwärmt. Daraufhin werden bei Raumtemperatur 0,4 g Kaliumhydroxid und 1 g (0,007 mol) 2-Chlorbenzaldehyd zugesetzt und 6 Stunden gerührt. Anschließend wird der Lösung Wasser (50 ml) zugesetzt und es wird mehrmals mit Methyl-tert.-butylether ausgeschüttelt. Die organische Phase wird mit Wasser gewaschen, über Natriumsulfat getrocknet und eingeengt. Der verbleibende Rückstand wird einer Chromatographie an Kieselgel mit n-Hexan/Essigester 9 : 1 unterworfen, wobei 0,9 g (39 %) cis-2-(1H-1,2,4-Triazol-1-yl-methyl)-2-(4-fluorphenyl)-3-(2-chlorphenyl)-o xiran erhalten werden.

## Patentansprüche

**1.** Verfahren zur Herstellung von cis-2-(1H-1,2,4-Triazol-1-yl-methyl)-2-(halogenphenyl)-3-(halogenphenyl)-oxiran der allgemeinen Formel 1

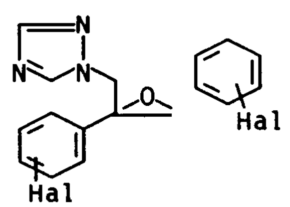

I,

in welcher Hal gleich oder verschieden ist und jeweils Fluor, Chlor oder Brom bedeutet, dadurch gekennzeichnet, daß man entweder

a) eine Carbonylverbindung der Formel II

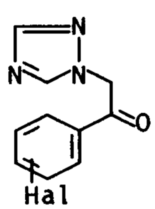

II

in welcher Hal für Fluor, Chlor und Brom steht, in Gegenwart einer Base mit einem Sulfoniumsalz der Formel VII

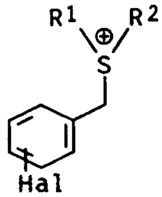

VII

in welcher Hal die oben angegebene Bedeutung hat und $R^1$ und $R^2$ unabhängig voneinander Alkyl, Cycloalkyl, einen Benzylrest, der im Phenylkern in gleicher Weise halogensubstituiert ist wie der bereits vorhandene Benzylrest in VII oder einen aromatischen Rest bedeuten, oder gemeinsam einen gesättigten oder ungesättigten 5- oder 6-gliedrigen Ring bilden, wobei das Sulfoniumsalz aus einem Benzylhalogenid der Formel III

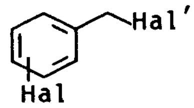

III,

in der Hal die o.g. Bedeutung hat und Hal' für Chlor, Brom oder Iod steht, und einem Sulfid der Formel IV

$R^1$-S-$R^2$ IV

gebildet wird, in Gegenwart oder Abwesenheit eines inerten Lösungsmittels zum Oxiran I umsetzt oder
b) einen Benzaldehyd der Formel VI

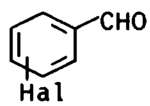

VI,

in der Hal die o.g. Bedeutung hat, in Gegenwart einer Base mit einem Sulfoniumsalz der Formel VIII

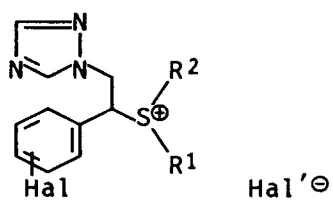

VIII,

in welcher Hal, Hal', $R^1$ und $R^2$ die o.g. Bedeutung haben, das aus dem entsprechenden Benzylhalogenidderivat der Formel V

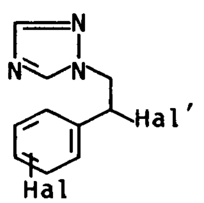

V,

in welcher Hal und Hal' jeweils die oben angegebene Bedeutung haben, und dem Sulfid IV gebildet wird, in Gegenwart oder Abwesenheit eines inerten Lösungsmittels zum Oxiran I umsetzt.

**2.** Verfahren nach Anspruch 1, dadurch gekennzeichnet, daß man die Umsetzungen a) bzw. b) ohne Isolierung der Sulfoniumsalze VII bzw. VIII durchführt.

**3.** Verfahren nach Anspruch 1, dadurch gekennzeichnet, daß man zunächst die Sulfoniumsalze VII bzw. VIII herstellt, isoliert und in einem zweiten Reaktionsschritt in Gegenwart einer Base mit den Carbonylverbindungen II bzw. VI zu den Oxiranen I umsetzt.

**4.** Verfahren nach Anspruch 1, dadurch gekennzeichnet, daß man ein Gemisch der Carbonylverbindung II und dem Benzylhalogenid III in einem Lösungsmittel zu einer Lösung des Sulfids IV in diesem Lösungsmittel in Gegenwart einer Base gibt.

**5.** Verfahren nach Anspruch 1, dadurch gekennzeichnet, daß man ein Gemisch der Carbonylverbindung VI und des Benzylhalogenidderivats V in einem Lösungsmittel zu einer Lösung des Sulfids IV in diesem Lösungsmittel in Gegenwart einer Base gibt.

**6.** Verfahren nach Anspruch 1, dadurch gekennzeichnet, daß man das Sulfoniumsalz VII in einem inerten Lösungsmittel mit der Base deprotoniert und anschließend die Carbonylverbindung II zusetzt.

**7.** Verfahren nach Anspruch 1, dadurch gekennzeichnet, daß man das Sulfid IV und das Benzylhalogenid V in einem inerten Lösungsmittel bei Temperaturen von 20 bis 100 °C umsetzt zum Sulfoniumsalz VIII, dieses deprotoniert und zuletzt dem Benzaldehyd VI zusetzt.

**8.** Verfahren nach Anspruch 1, dadurch gekennzeichnet, daß man die Umsetzung des Sulfids IV mit dem Benzylhalogenid V in Gegenwart von Schwermetallsalzen vornimmt.

**9.** Verfahren nach Anspruch 1, dadurch gekennzeichnet, daß man die Deprotonierung der Sulfoniumsalze VII bvzw. VIII bei Temperaturen von -30 °C bis +50 °C vornimmt.

**10.** Verfahren nach Anspruch 1, dadurch gekennzeichnet, daß man die Herstellung der Sulfoniumsalze VII bzw. VIII bei Temperaturen von 0 bis 120 °C vornimmt.